# EUROPEAN PATENT APPLICATION

(11) **EP 2 881 057 A1**
(43) Date of publication of application: **10.06.2015**
(21) Application number: 13195791.2
(22) Date of filing: 05.12.2013
(51) Int. Cl.: A61B 18/14, A61M 25/01

(54) **Apparatus for creating linear lesions in body tissue within a body vessel**

(71) Applicant: Region Nordjylland, 9220 Aalborg Øst (DK)
(72) Inventor: Jensen, Svend Eggert, 8382 Hinnerup (DK); Hansen, Steen Møller, 8541 Skødstrup (DK)
(74) Representative: Høiberg A/S

(57) **Abstract**

The invention regards an apparatus for creating linear lesions in body tissue within a body vessel comprising a catheter adapted for insertion into the body vessel, the catheter having a proximal end and a distal end, a loop of a non-conducting material at the distal end being deformable such that when the loop is pressed against a wall of the body vessel a section of the loop will conform to the internal contours of the wall of the body vessel, an linear ablating conductor disposed on the loop, such that when the loop is pressed against the wall of the body vessel a portion of the ablating conductor is in contact with the body tissue in a smaller area than the total area of the section of the loop conformed to the internal contours of the wall of the body vessel.

## Description

### Field of invention

The invention relates to an apparatus for creating linear lesions in body tissue within a body vessel.

### Background of the Invention

Atrial fibrillation is an arrhythmic disorder where abnormal electrical signals are generated in the endocardial tissue causing irregular heart rhythm. Patients suffering from atrial fibrillation may, among other symptoms, have a feeling of skipped heart beats, chest discomfort, weakness and fatigue often resulting in reduced level of bodily function. Atrial fibrillation results in risk of systemic embolism and thereby stroke.

Interventional cardiologists have for many years used Radio Frequency Ablation in the treatment of atrial fibrillation. The treatment involves the attempt to isolate the pulmonary veins in the left atrium from the remaining electric conduction system of the heart. Such that the electrical signals generated in the endocardial tissue are blocked from affecting the heart rhythm.

Unfortunately the results of Radio Frequency Ablation have not been totally successful. The electric isolation often seems to be right after the procedure. However, the procedure results in an oedema of the tissue secondary to the ablation procedure and when the oedema disappears after a couple of weeks post-procedure the patient may experience new onset of atrial fibrillation. This is due to an incomplete isolation of the pulmonary veins in the left atrium from the remaining electric conduction system of the heart.

Devices for performing ablation of body tissue are known in the art, such as US 6,190,382 which discloses a radio-frequency catheter system for ablating biological tissues of a body vessel in a patient including a catheter, a deployable antenna guide disposed at the distal portion of the catheter and a radio-frequency antenna mounted on the antenna guide. The radio-frequency antenna includes a helical coil which defines an axial passageway to accommodate the antenna guide, and is adapted to receive and transmit radio-frequency energy for tissue ablation. Upon deployment, the antenna guide acquires a loop configuration which establishes line contact with the body vessel conformable to its internal contour to prescribe the precise and affixed tissue ablation pathway despite body vessel movements. The radio-frequency antenna is carried by the antenna guide to be deployed along the established tissue ablation pathway.

Further, WO 97/32525 discloses an apparatus for ablating body tissue, and particularly for creating linear lesions within a chamber of a patient's heart, includes an elongate member having an ablation section. The ablation section includes an infusion tube and a plurality of spaced electrodes. The infusion tube and electrodes are covered by a fluid permeable foam material, and the foam material is covered by a fluid impermeable covering having a plurality of holes formed in it. During use, the ablation section is positioned against tissue to be ablated. Radiofrequency energy is delivered to the electrodes while saline or other conductive fluid is delivered to the infusion tube. The fluid exits the infusion tube at the ablation section, contacts the electrodes, and carries radio-frequency energy from the electrodes through the foam, through the holes in the covering and into contact with the body tissue to form a burn in the body tissue

Among the problems of the prior art are the difficulty in controlling the ablation with high precision. In use the prior arts ablating conductor will change shape and position during the ablation process rendering it difficult to control depth and size of the lesions.

### Summary of the invention

Considering the prior art described above, it is an object of the present invention to provide an apparatus for creating precise linear lesions in body tissue within a body vessel.

The object can be achieved by means of an apparatus for creating linear lesions in body tissue within a body vessel comprising, a catheter adapted for insertion into the body vessel, the catheter having a proximal end and a distal end, a loop of a non-conducting material at the distal end being deformable such that when the loop is pressed against a wall of the body vessel a section of the loop will conform to the internal contours of the wall of the body vessel, a linear ablating conductor disposed on the loop, such that when the loop is pressed against the wall of the body vessel a portion of the ablating conductor is in contact with the body tissue in a smaller area than the total area of the section of the loop conformed to the internal contours of the wall of the body vessel.

Thus, it is possible to produce a linear lesion with high precision. When the loop of the present invention is pressed against the wall of the body vessel to be ablated it is not only the ablating conductor but also the non-conducting material of the loop that is in contact with the wall. This is because the portion of the ablating conductor in contact with the body tissue is smaller than the total area of the section of the loop conformed to the internal contours of the wall of the body vessel. Thus, the force applied to the wall is so to speak distributed to both the linear ablating conductor and the loop of a non-conducting material. The position and the deformation of the ablating conductor will then, to some extend, be controlled by the section of the loop conformed to the internal contours of the wall of the body vessel which is not covered by the ablating conductor.

Body vessel is to be understood as a cavity, duct or vessel within the body. When treating atrial fibrillation the body vessel is the cardiac lumen and the body tissue to be ablated is the myocardial wall.

It is to be understood that when the ablating conductor is in contact with the body tissue, it means that the ablating conductor is in thermodynamic contact so that the heat generated by the ablating conductor can ablate the body tissue. Conform can be understood as adapt; such that when the loop is pressed against a wall of the body vessel a section of the loop will adapt to the internal contours of the wall of the body vessel.

By use of the present invention it is possible to create a linear ablation as the net sum of a plurality of linear lesions.

The ablating conductor can be supplied with current in order to heat it, this can for example be a high frequency alternating current, such as 350-500 kHz; this is known in the art as radio frequency ablation. The advantage of using high frequency alternating current compared to low frequency AC or pulses of DC is, that it does not directly stimulate nerves or heart muscle. The current can be delivered by use of conductors in the catheter.

A loop of a non-conducting material can be a thin strip of flexible material, for example a band of silicone, rubber or a plastic material. The use of non-conducting material ensures an insulation of the ablating conductor. The loop can be in the form of a band whereon the linear ablating conductor is arranged in the entire length of the loop. Preferably, the width of the band is greater than the width of the linear ablating conductor.

In an embodiment, the catheter comprises a distal opening at the distal end and a lumen extending from the proximal end to the distal end wherein, the loop is adapted to be arranged within the lumen and deployable beyond the opening of the distal end. Thus, it is possible to unfold the loop and the ablating part of the apparatus when the catheter is in the correct position; this eases the insertion of the catheter.

Advantageously, a plurality of ablating conductors disposed on the loop. Thus, it is possible to dissipate more energy to the body tissue.

In an embodiment, a plurality of impedance measuring conductors are disposed on the loop, such that when the guide loop is pressed against the wall of the body vessel the plurality of impedance measuring conductors make contact with the wall of the body vessel. By measuring the impedance between impedance measuring conductors, it is possible to ensure that the loop is in contact with the wall of the body vessel.

Preferably, the loop is in the form of a band whereon the plurality of impedance measuring conductor are arranged in the entire length of the loop and each impedance measuring conductor are insulated except in one point where the impedance measuring conductor is suitable for making contact with the wall of the body vessel. Points are to be understood as a small portion of the impedance measuring conductors, that are non-insulated such that a impedance measurement between two points can determine if the points are in contact with the body vessel and consequently if the ablating conductor is in contact with the body vessel. In this way it is possible to divide the loop and it can be tested if those segments have contact with the wall of the body vessel.

In an embodiment, the portion of the ablating conductor which is in contact with the body tissue has a length greater than 5 mm. Thus, it is possible to make a long linear ablation with only a few linear lesions.

Advantageously, the apparatus further comprises a tube at the distal end suitable for irrigation of the body tissue within the body vessel. Thus, it is, for example, possible to provide further cooling in order to prevent damages to surrounding tissue and/or body fluids.

In an embodiment, the area of the ablating conductor in contact with the body tissue is smaller than the area of the non-conducting material of the loop in contact with the body tissue. Hereby, control of the depth and size of the linear lesions is improved even further. Preferably, the area of the ablating conductor in contact with the body tissue is at least two times smaller than the area of the non-conducting material of the loop in contact with the body tissue.

In an embodiment the loop further comprises a temperature measuring conductor. This conductor can be used to determine the temperature of the wall of the body vessel. This can for example be done by measuring the change in conductivity of a temperature measuring conductor which is based on a change in temperature.

In an embodiment the loop further comprises an electrocardiographic (ECG) conductor, suitable for determining the electrical activity of the heart over time.

In an embodiment, the portion of the linear ablating conductor not in contact with the body tissue when the loop is pressed against the wall, is at least partly covered by an insulating material. Preferably, the portion of the linear ablating conductor is not in contact with the body tissue when the loop is pressed against the wall, and is covered in its entirety by an insulating material. Insulating the part of the ablating conductor that is not in contact with the body tissue minimizes the energy released to the surrounding tissue and/or bodily fluid. When treating atrial fibrillation the loop is positioned in the cardiac lumen which is filled with blood. When heating the blood there is a high risk of coagulation and consequently the production of an embolism which in some instances can lead to stroke. Therefore, it is highly desirable to minimize the energy released to surrounding fluids in order to minimize the side effects of ablating procedure. In addition, by limiting the energy released to the surrounding the energy loss is minimized resulting in an increased ablating capability of the ablating conductor in contact with the body tissue. Thus, the energy delivered to the conductor can be lowered if the same linear lesion is desired or a larger linear lesion can be created if the energy delivered to the conductor is kept at the same level.

The feature of, at least partly, insulating the portion of the ablating conductor that is not in contact with the body tissue to be ablated can advantageously be used for all apparatuses for creating lesions in body tissue within a body vessel. This can be defined in the following item:
An apparatus for creating lesions in body tissue within a body vessel comprising, a catheter adapted for insertion into the body vessel, the catheter having a proximal end and a distal end with a distal opening and a lumen extending from the proximal end to the distal end, an ablating conductor at the distal adapted to be pressed against a wall of the body vessel, such that a first portion of the ablating conductor is in contact with the body tissue and a second portion of the ablating conductor is not in contact with the body tissue wherein the second portion of the ablating conductor is at least partly covered by an insulating material. Preferably, the second portion of the ablating conductor is covered in its entirety by an insulating material.

An apparatus of the abovementioned type has the advantages as mentioned above. Further it is to be understood that it can be modified by any of the features presented in this document.

The invention also regards a method for ablating for creating linear lesions in body tissue within a body vessel, comprising the steps of, providing a catheter having a proximal end and a distal end with a loop of a non-conducting and deformable material at the distal end, manipulating the distal end of the catheter through the body and press the loop against a wall of the body vessel such that it conforms to the internal contours of the wall of the body vessel, supply energy to an ablating conductor disposed on the loop wherein the area of the ablating conductor in contact with the wall of the body vessel is smaller than the area of the loop in contact with the wall of the body vessel.

It is to be understood that in embodiments of the method it can be adapted to use any of the preferred embodiments of the apparatus mentioned in the present document.

### Description of the drawings

The invention will in the following be described in greater detail with reference to the accompanying drawings:
- Fig. 1: a schematic side view of an embodiment of the invention when arranged within a lumen and when deployed.
- Fig. 2: a schematic side view of an embodiment of the invention when close to and when pressed against a wall of a body vessel.
- Fig. 3: a schematic view of a loop according to an embodiment of the invention.
- Fig. 4: a schematic view of a loop according to an embodiment of the invention.
- Fig. 5: a schematic side view of an embodiment of the invention when pressed against a wall of a body vessel.
- Fig. 6: a schematic side view of an embodiment of the invention.
- Fig. 7: a schematic view of linear lesions performed by an embodiment of the invention.

### Detailed description of the invention

In the following embodiments of the invention for treating atrial fibrillation is presented. Radio frequency energy is thus delivered to the ablating conductor in order to generate energy needed for ablating. The catheter is manoeuvred to the left atria where lesions in the myocardial wall (the wall of the body vessel) are created in order to isolate pulmonary veins in the left atrium from the remaining electric conduction system of the heart and thus blocking the excess electrical signals generated in the endocardial tissue from affecting the heart rhythm.

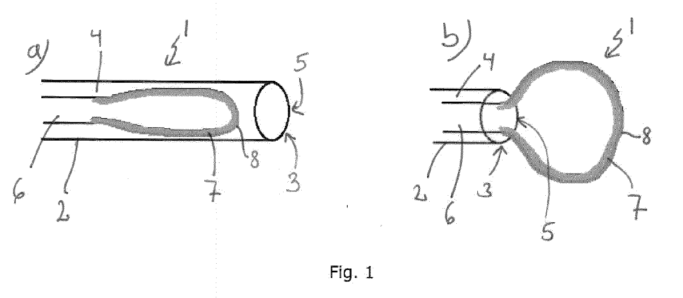

Fig. 1 shows an apparatus 1 for creating linear lesions in body tissue, having a catheter 2 with a distal end 3. The proximal end is not shown in the figures. The catheter 2 has a lumen 4 and an opening 5 in the distal end. Within the lumen 4 a guide 6 is shown which is connected to the loop 7.

In fig. 1a the loop 7 is shown in an unfolded configuration where it is arranged within the lumen 4, in this configuration the catheter 2 can be inserted into the human body and manoeuvred to the desired body vessel. In the present embodiment, the catheter 2 is inserted through the femoral artery and manoeuvred to the left atria for ablating the myocardial wall 9. The procedures and devices needed for manoeuvring the catheter 2 within the human body is known in the art and will not be explained further.

When the distal end 3 of the catheter 2 is adjacent to the myocardial wall 9 the loop 7 is deployed or unfolded, as shown in fig. 1 b. The loop 7 can be made of a band of non-conducting material such as silicone. The band can be 3 mm wide and 14 mm in length. The loop 7 is sufficiently flexible to enable it to be arranged with in the lumen 4 as shown in fig. 1a and rigidly enough so that when deployed it can have a circular like form as seen on fig. 1 b.

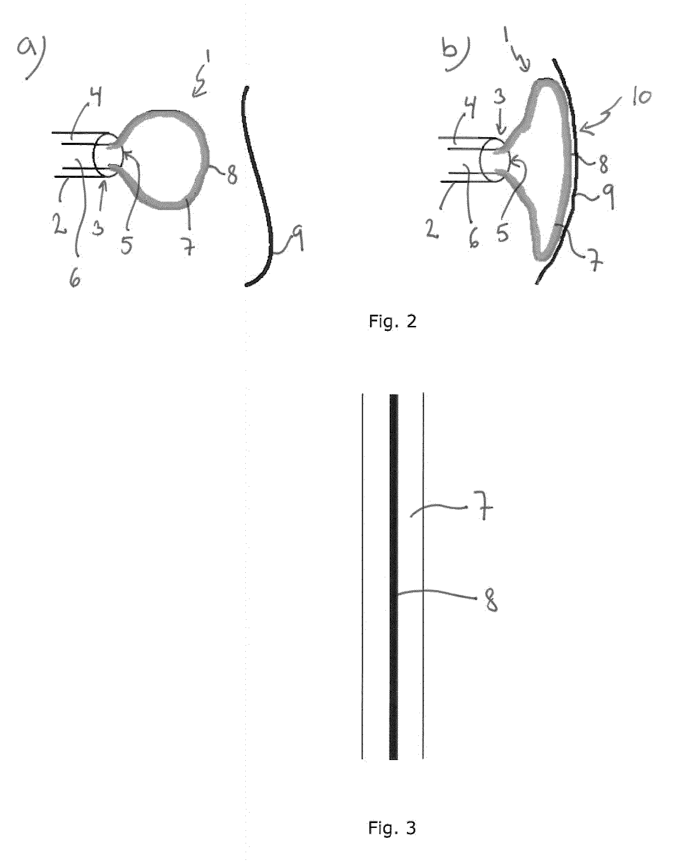

Fig. 2 shows a schematic view of how the loop 7 conforms to the myocardial wall 9. Fig 2a shows the catheter 2 in an unfolded configuration with the loop 7 deployed beyond the opening 5 of the distal end 3. The myocardial wall 9 is not yet exposed to the pressure from the loop which most likely will deform the wall 9. When the loop 7 is pressed against the myocardial wall 9, as shown in fig. 2b, both the loop 7 and the wall 9 deforms for optimal alignment and contact there between. Accordingly, a section 10 of the loop 7 is conformed to the internal contours of the myocardial wall 9 for optimal contact between the loop 7 and the wall 9. The length of the section 10 is defined by the length of the loop 7 and can have a length approx. between 5 and 9 mm, preferably approx. 7mm.

Fig. 3 discloses an embodiment of a section of a loop 7 where an ablating conductor 8 is disposed on the loop 7. The ablating conductor 8 is in the form of a band that is attached to the non-conducting material of the loop 7. The loop 7 is wider than the ablating conductor so that the stress on the wall of the body vessel 9 is minimized. When the loop 7 is in contact with the wall 9 it is possible to produce a linear lesion in the body tissue of the wall 9 by applying a current to the ablating conductor 8. The depth of the linear lesion is primarily defined by the energy delivered to the conductor and only secondary by the pressure applied to the wall 9.

When the loop 7 in the form of a band and with a ablating conductor 8 as shown in fig 3 are used, the loop 7 will not dig into the lesion during the ablation because the non-conducting part of the loop 7 (which is not covered by the ablating conductor 8) will hinder that the ablating conductor 8 is pressed into the lesion while ablating. The non-conducting part of the loop 7, that are at each side of the ablating conductor 8, will so to speak rest on the sides of the linear lesions and thus keep the loop 7 from changing shape during ablation. The depth of the lesions can then be defined very accurately from the radio frequency energy supplied to the ablating conductor 8.

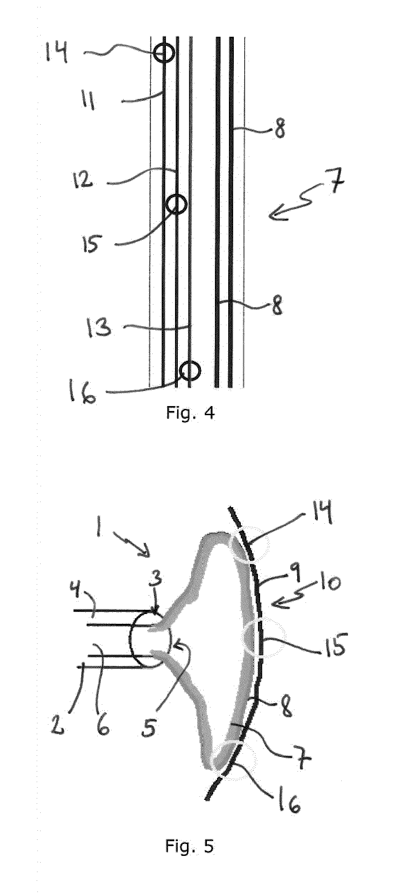

Fig 4 discloses an alternative embodiment of a loop 7. Here, two ablating conductors 8 are disposed on the loop 7. The embodiment can also be modified to have only one ablating conductor 8, or alternatively have any number of ablating conductors 8. In similar fashion; the loop 7 shown in fig. 3 could have any number of ablating conductors 8. In fig. 4 the loop 7 also has three impedance measuring conductor, a first impedance measuring conductor 11, a second impedance measuring conductor 12 and a third impedance measuring conductor 13. The conductors are insulated except in a first 14, second 15 and third 16 point. The points 14, 15, 16 can be a small portions of the impedance measuring conductor 11, 12 13 which is not covered by insulation. Each of the impedance measuring conductors 11, 12 13 are in contact with the myocardial wall 9 at their respective points 14, 15, 16 when the loop is pressed against it, as seen on fig. 5. Accordingly, it is possible to measure the impedance between the first point 14 and the second point 15 this measurement will indicate if the points 14 and 15 are in contact with the wall 9 and consequently indicate if the loop 7, and thus the ablating conductor 8 is positioned correctly on the wall 9 in the portion between the points 14 and 15. In similar fashion it is possible to ensure that the loop 7 and thus if the ablating conductor 8 is positioned correctly on the wall 9 in the portion between the points 15 and 16.

It is to be understood that the loop 7 can have any number of impedance measuring conductors and thus divide the the section 10 of the loop 7 conformed to the myocardial wall 9 into small portions wherein it can be ensured if each portion have contact with the wall 9.

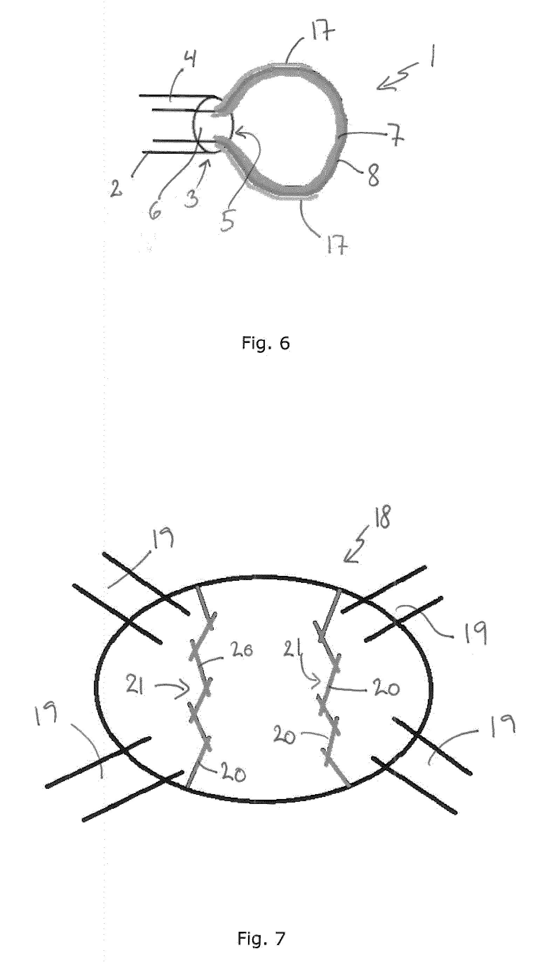

In addition to the conductor disposed on the loop 7 shown in fig. 3 and 4 the loop can comprise further conductors. Fig. 6 discloses a further advantageous embodiment of the present invention. The figure is similar to fig. 1b but the loop further comprises an insulating layer 17 disposed on the loop 7. It is disposed such that it covers a part of the ablating conductor 8, as shown on fig. 6 the insulating layer 17 covers both of the parts of the loop 7 runs from the catheter to the section 10 of the loop 7 that conforms to the internal contours of the myocardial wall 9. The insulating layer 17 can be made of silicone or another insulating material. It protects the surrounding tissue and body fluids and directs ablating energy to the tissue to be ablated; in case of ablation of atrial fibrillation it protects the myocardial wall from unintended lesions and blood from coagulation which can lead to the formation of an embolus and stroke.

The insulating layer 17 preferably also covers a small part of the ablating conductor 8 that conforms otherwise would be in contact with the myocardial wall 9 hereby it is ensured that the ablating conductor 8 does not get in contact with anything but the myocardial wall 9.

Fig. 7 shows schematic view of the left atria 18 with pulmonary veins 19. When treating atrial fibrillation embodiments of the present invention can make linear lesions 20 in the pattern 21. The linear lesions 20 overlaps such that a full isolation of the pulmonary veins in the left atrium 18 from the remaining electric conduction system of the heart pattern.

In order to make linear lesions 20 in the pattern 21 it is advantageously if the loop 7 can be rotated in relation to the catheter 2. This can for example be enabled by use of a guide 6 that can rotate within the catheter 2, which will enable the angling of the loop 7. During an ablating procedure where a pattern 21 as seen in fig 7 is desired a first linear lesion can be made, subsequently the catheter 2 can be moved to a new position and the loop 7 angled such that the second linear lesion overlaps with the first linear lesion. Any number of further overlapping linear lesions 20 can be made in similar fashion. In fig. 7 the pattern 21 comprises 6 linear lesions 20. In this fashion it is possible to ensure that the pattern 21 forms an unbroken line of linear lesions 20.

### Reference list:

- 1: apparatus
- 2: catheter
- 3: distal end
- 4: lumen
- 5: opening
- 6: guide
- 7: loop
- 8: ablating conductor
- 9: myocardial wall
- 10: section
- 11: first impedance measuring conductor
- 12: second impedance measuring conductor
- 13: third impedance measuring conductor
- 14: first point
- 15: second point
- 16: third point
- 17: insulating layer
- 18: left atrium
- 19: pulmonary veins
- 20: linear lesions
- 21: pattern

## Claims

1. Apparatus for creating linear lesions in body tissue within a body vessel comprising,
- a catheter adapted for insertion into the body vessel, the catheter having a proximal end and a distal end,
- a loop of a non-conducting material at the distal end being deformable such that when the loop is pressed against a wall of the body vessel a section of the loop will conform to the internal contours of the wall of the body vessel,
- an linear ablating conductor disposed on the loop, such that when the loop is pressed against the wall of the body vessel a portion of the ablating conductor is in contact with the body tissue in a smaller area than the total area of the section of the loop conformed to the internal contours of the wall of the body vessel.

2. Apparatus according to claim 1, wherein the loop is in the form of a band whereon the linear ablating conductor is arranged in the entire length of the loop.

3. Apparatus according to claim 2, wherein the width of the band is greater than the width of the linear ablating conductor.

4. Apparatus according to any of the preceding claims, wherein the catheter comprises a distal opening at the distal end and a lumen extending from the proximal end to the distal end, and wherein the loop is adapted to be arranged within the lumen and deployable beyond the opening of the distal end.

5. Apparatus according to any of the preceding claims, wherein a plurality of ablating conductors disposed on the loop.

6. Apparatus according to any of the preceding claims, wherein a plurality of impedance measuring conductors are disposed on the loop, such that when the guide loop is pressed against the wall of the body vessel the plurality of impedance measuring conductors make contact with the wall of the body vessel.

7. Apparatus according to claim 6, wherein the loop is in the form of a band whereon the plurality of impedance measuring conductor are arranged in the entire length of the loop and each impedance measuring conductor are insulated except in one point where the impedance measuring conductor is suitable for making contact with the wall of the body vessel.

8. Apparatus according to any of the preceding claims, wherein the portion of the ablating conductor which is in contact with the body tissue has a length greater than 5 mm.

9. Apparatus according to any of the preceding claims, wherein the apparatus further comprises a tube at the distal end suitable for irrigation of the body tissue within the body vessel.

10. Apparatus according to any of the preceding claims, wherein the area of the ablating conductor in contact with the body tissue is smaller than the area of the non-conducting material of the loop in contact with the body tissue.

11. Apparatus according to claim 10, wherein the area of the ablating conductor in contact with the body tissue is at least two times smaller than the area of the non-conducting material of the loop in contact with the body tissue.

12. Apparatus according to any of the preceding claims, wherein the portion of the linear ablating conductor not in contact with the body tissue when the loop is pressed against the wall, is at least partly covered by an insulating material.

13. Method for ablating for creating linear lesions in body tissue within a body vessel, comprising the steps of,
- providing a catheter having a proximal end and a distal end with a loop of a non-conducting and deformable material at the distal end,
- manipulating the distal end of the catheter through the body and press the loop against a wall of the body vessel such that it conforms to the internal contours of the wall of the body vessel,
- supply energy to an ablating conductor disposed on the loop wherein the area of the ablating conductor in contact with the wall of the body vessel is smaller than the area of the loop in contact with the wall of the body vessel.
